# EUROPEAN PATENT APPLICATION

(11) **EP 3 501 514 A1**
(43) Date of publication of application: **26.06.2019**
(21) Application number: 17306889.1
(22) Date of filing: 21.12.2017
(51) Int. Cl.: A61K 31/17, A61P 9/00, A61P 43/00

(54) **USE OF CELIPROLOL FOR TREATING VASCULAR EHLERS-DANLOS SYNDROME IN WOMEN DURING PREGNANCY AND PERIPARTUM PERIOD**

(71) Applicant: Assistance Publique-Hôpitaux de Paris (AP-HP), 75004 Paris (FR); UNIVERSITE PARIS DESCARTES, 75006 Paris (FR)
(72) Inventor: FRANK, Michael, 78950 GAMBAIS (FR); JEUNEMAITRE, Xavier, 75012 PARIS (FR); BENACHI, Alexandra, 75007 PARIS (FR)
(74) Representative: Santarelli

(57) **Abstract**

The present invention relates to the use of celiprolol or a pharmaceutically acceptable salt thereof for treating vascular Ehlers-Danlos syndrome in women during pregnancy and peripartum period.

## Description

### FIELD OF THE INVENTION

The present invention relates to the prevention of cardiovascular, gastrointestinal and obstetrical events during pregnancy, delivery, and peripartum period in women suffering from an orphan disease, namely vascular Ehlers-Danlos syndrome (vEDS). More specifically, the invention pertains to the use of celiprolol for treating vEDS in women during pregnancy and during the peripartum period.

### BACKGROUND OF THE INVENTION

Vascular Ehlers-Danlos syndrome (OMIM#13050; EDS vascular type) is a rare genetic disease caused by mutations in the COL3A1 gene encoding type III procollagen. Its evolution is characterized by recurrent, unpredictable and potentially life-threatening arterial, gastrointestinal and obstetrical accidents or events in young adults [1, 2]. In women, pregnancy and more particularly delivery and the peripartum period are associated with increased risk of uterine and/or arterial rupture, which have been associated with maternal death [3-5]. Case-series and small patient cohorts initially proposed maternal mortality rates ranging from 25 to 38.5% [6, 7], which were updated in the year 2000's to 11.5% in a large patient cohort [1]. More recently, these estimates have been further revised to a lower mortality rate of 5.3% [8].

Other complications, such as premature rupture of membranes, preterm labour and prematurity can also occur in this patient population, but have been extensively documented in patients without taking into account the specific subtypes of Ehlers-Danlos syndromes (EDS). These data also often relied on patient surveys without medical interview [9, 10]. Finally, it is not known whether maternal morbidity and mortality of vascular EDS may be improved by specific care strategies. Programmed caesarean section (c-section) has been suggested to improve vEDS patient outcome, but without formal proof of efficacy nor any evaluation of neonatal outcome [7]. Consequently, contraindication of pregnancy in vascular EDS has been discussed recurrently and has been subject of a formal recommendation [7, 11].

In 2010, Ong *et al.* reported the results of a clinical trial study for assessing the effects of celiprolol on prevention of cardiovascular events in vascular Ehlers-Danlos syndrome (BBEST study). However, pregnant women and women likely to become pregnant were considered as ineligible for enrollment in this study because of the potential risks of celiprolol treatment for the fetus. The adverse effects on the fetus that were anticipated and thus led to the exclusion of pregnant women in the BBEST study were intrauterine growth retardation, hypotonicity and neonatal hypoglycaemia.

Thus there remains an unmet medical need for novel, appropriate and effective treatment of women with vEDS during pregnancy, delivery and the peripartum (or perinatal) period.

### SUMMARY OF THE INVENTION

The present invention provides, *inter alia,* methods of treating vEDS in women during pregnancy, delivery and the peripartum period by administering to such women celiprolol or a pharmaceutically acceptable salt thereof. The present invention discloses that such treatment efficiently and effectively protected these women from cardiovascular, gastrointestinal and obstetrical accidents and events, and surprisingly, that such treatment did not have any of the expected adverse effects on the fetus.

The present invention further provides for the use of celiprolol or a pharmaceutically acceptable salt thereof for preventing cardiovascular, obstetrical and gastrointestinal events in vEDS women during pregnancy, delivery and the perinatal or peripartum period.

In one embodiment, the present invention provides a method for treating vEDS in a woman during pregnancy, during delivery, or during the peripartum period, the method comprising administering to the woman celiprolol or a pharmaceutically acceptable salt thereof, thereby treating vEDS in the woman. In some embodiments, celiprolol, or a pharmaceutically acceptable salt thereof, is administered to the woman at a dosage of at least 100 mg per day. In some embodiments, celiprolol, or a pharmaceutically acceptable salt thereof, is administered to the woman at a dosage ranging from about 200 mg to about 600 mg per day; from about 200 mg to about 500 mg per day; or from about 200 mg to about 600 mg per day. In some embodiments, the daily dosage of celiprolol, or a pharmaceutically acceptable salt thereof, is obtained by administering celiprolol, or a pharmaceutically acceptable salt thereof, to the women once daily, twice daily, or thrice daily.

In some embodiments, the present invention provides a method for treating woman having vEDS during pregnancy, during delivery, or during the peripartum period by administering or providing celiprolol, or a pharmaceutically acceptable salt, to the woman, wherein the method prevents or reduces cardiovascular, gastrointestinal, or obstetrical events. In some embodiments, the present invention provides a method for treating woman having vEDS during pregnancy, during delivery, or during the peripartum period with celiprolol, or a pharmaceutically acceptable salt, wherein the method prevents or reduces the risk of arterial dissection or arterial rupture; prevents or reduces the risk of obstetrical accidents; prevents or reduces the risk of uterine rupture; or prevents or reduces the risk of pregnancy-related or pregnancy-associated death. In some embodiments, the present invention provides a method for treating a woman having vEDS, wherein the treatment is during pregnancy, during delivery, or during the peripartum period, wherein the method comprises administering to the woman celiprolol, or a pharmaceutically acceptable salt thereof, wherein the method prevents or reduces the risk of death following termination of pregnancy, or prevents or reduces the risk of preterm birth.

### BRIEF DESCRIPTION OF THE DRAWINGS

**Figure 1****:** Maternal mortality analysis, flow chart.
**Figure 2****:** Number of live-born, maternal deaths and surviving parturients, by generation.
**Figure 3**: Birth weight (A) and size (B) as a function of gestational age (boys and girls).

### DETAILED DESCRIPTION OF THE INVENTION

The present invention provides a method of treating vascular Ehlers-Danlos syndrome in a woman during pregnancy, during delivery and during the peripartum period, the method comprising administering celiprolol, or a pharmaceutically acceptable salt thereof, to the woman.

In one embodiment of the present invention, a method is provided for treating vEDS in a woman during pregnancy, during delivery, or during the peripartum period, the method comprising administering to the woman celiprolol, or a pharmaceutically acceptable salt thereof, thereby treating vEDS in the woman during pregnancy, during delivery, or during the peripartum period. In some embodiments, celiprolol, or a pharmaceutically acceptable salt thereof, is administered to the woman at a dosage of at least 100 mg per day. In some embodiments, celiprolol, or a pharmaceutically acceptable salt thereof, is administered to the woman at a dosage ranging from about 200 mg to about 600 mg per day; from about 200 mg to about 500 mg per day; from about 200 mg to about 400 mg per day. In some embodiments, celiprolol, or a pharmaceutically acceptable salt thereof, is administered to the woman at a dosage of about 100 mg per day, at a dosage of about 200 mg per day, at a dosage of about 300 mg per day, at a dosage of about 400 mg per day, at a dosage of about 500 mg per day, or at a dosage of about 600 mg per day. In some embodiments, the daily dosage of celiprolol, or a pharmaceutically acceptable salt thereof, is obtained by administering celiprolol, or a pharmaceutically acceptable salt thereof, to the women once daily, twice daily, or thrice daily.

In some embodiments, treatment of woman having vEDS during pregnancy, during delivery, or during the peripartum period with celiprolol, or a pharmaceutically acceptable salt thereof, according to the methods of the present invention, prevents or reduces cardiovascular, gastrointestinal, or obstetrical events. In some embodiments, treatment of woman having vEDS during pregnancy, during delivery, or during the peripartum period with celiprolol, or a pharmaceutically acceptable salt thereof, according to the methods of the present invention, prevents or reduces the risk of arterial dissection or arterial rupture; prevents or reduces the risk of obstetrical accidents; prevents or reduces the risk of uterine rupture; or prevents or reduces the risk of pregnancy-related or pregnancy-associated death. In some embodiments, treatment of woman having vEDS during pregnancy, during delivery, or during the peripartum period with celiprolol, or a pharmaceutically acceptable salt thereof, according to the methods of the present invention, prevents or reduces the risk of death following termination of pregnancy, or prevents or reduces the risk of preterm birth.

As used herein, the terms "treat", "treatment" and "treating" refer to any reduction of one or more symptom(s) associated with vascular Ehlers-Danlos syndrome; for example, reduction of the occurrence and/or severity of cardiovascular and/or obstetrical accidents, and/or a decrease of the risk of maternal death during pregnancy and post-partum period that results from the administration of celiprolol alone or combined with one or more other therapies or care of these patients.

Celiprolol (brand names Cardem®, Selectol®, Celipres®, Celipro®, Celol®, Cordiax®, Dilanorm®) is a medication in the class of beta blockers. Its chemical formula is N'-(3-Acetyl-4-(3-((1,1-dimethylethyl)amino)-2-hydroxypropoxy)phenyl)-N, N-diethylurea, its CAS number is 56980-93-9 and Drug Bank number is DB04846.

In the present text, the peripartum period is defined as the last month of pregnancy to five months postpartum.

According to one embodiment, the present invention provides a method of treating vascular Ehlers-Danlos syndrome in a woman during pregnancy, during delivery, or during the peripartum period, the method comprising administering celiprolol, or a pharmaceutically acceptable salt thereof, to a woman in need thereof, wherein treatment begins with a titration period wherein celiprolol, or a pharmaceutically acceptable salt thereof, is administered at a dosage of 100 mg daily during one month and increased by steps of 100 mg/day every month over a 3-month period to reach a dosage of 400 mg per day (or at least 400 mg per day).

In other embodiments, a dose escalation regimen is used for such treatment. In the dose escalation regimen provided herein, the first daily dosage is about 100 mg during the initial month, the initial 30 days, or the initial 28 days; the second daily dosage is about 200 mg (e.g., 200 mg once daily, 100 mg twice-daily) during the second month, the second 30 days, or the second 28 days; the third daily dosage is about 300 mg (e.g., 300 mg once daily, 150 mg twice daily, 100 mg thrice daily) during the third month; and the fourth daily dosage is about 400 mg daily (e.g., 400 mg once daily, 200 mg twice daily). Methods of the present invention can further comprise additional steps after the three-month dose escalation/up-titration period, to further increase the daily dose of celiprolol, or a pharmaceutically acceptable salt thereof, administered to the woman. According to a particular embodiment, one or two additional dosage increases are performed after the three-month dose escalation/up-titration period, to reach a daily dosage of greater than 400 mg, such as for example, 500 mg or 600 mg of celiprolol, or a pharmaceutically acceptable salt thereof.

In some embodiments, the celiprolol or pharmaceutically acceptable salt thereof is celiprolol hydrochloride.

In some embodiments, the method is initiated as soon as pregnancy is confirmed, or soon thereafter. In other embodiments, the method is initiated soon after the woman gives birth. In some embodiments, treating woman according to one of the methods provided herein occurs during pregnancy and continues into the peripartum period.

In some embodiments, the woman is diagnosed with vEDS based on a phenotype of vEDS, or based on a molecular test vEDS (e.g., the woman is determined to have vEDS based on one or more genetic tests such as a test that determines that the subject has a glycine substitution within the triple helix or a splice-site variant within COL3A1 gene).

In some embodiments, the woman has a glycine substitution within the triple helix or a splice-site variant within COL3A1 gene. In some embodiments, the woman has previously had an acute vEDS-related event (e.g., an arterial event such as a rupture or dissection) prior to the initial dose of celiprolol, or a pharmaceutically acceptable salt thereof.

Other characteristics of the invention will also become apparent in the course of the description which follows of the biological assays which have been performed in the framework of the invention and which provide it with the required experimental support, without limiting its scope.

### EXAMPLES

The inventors herein report the analysis of a French cohort of vascular EDS patients. They specifically analyzed the issues and complications of pregnancies in women with molecularly proven vascular EDS or retrospectively obligate carriers of COL3A1 genetic variants. This analysis provides recent and precise estimates of maternal mortality and morbidity in this condition. The authors also describe the successful use of celiprolol for preventing cardiovascular and/or digestive and/or obstetrical events in women or during pregnancy, delivery and during the peripartum period.

### Methods

### Study population

For the mortality analysis, the medical charts and pedigrees of all patients known to the French referral center for rare vascular diseases (AP-HP, *Centre de Référence des Maladies Vasculaires Rares,* Hôpital Européen Georges Pompidou, Paris, France) for having a molecularly confirmed vascular EDS, were screened for past or ongoing pregnancies, and for maternal deaths in probands and pedigrees. All families known to our center with at least one proband diagnosed with vascular EDS (nationwide patient cohort) were considered for participation to the maternal mortality analysis after exclusion of ascendants of patients with neomutations (see below). Patients with a personal history of pregnancy were considered for participation to the obstetrical interview study.

### Maternal mortality analysis

To be eligible to the mortality analysis, families with one affected proband were to have a complete medical record for at least the index case, and a complete pedigree made by a genetic counsellor over two generations showing numbers of siblings, ascendants (aunts and uncles, grand-fathers and grand-mothers), number of life- and still-born children for each child-bearing woman, their age and cause of death when applicable.

In order to identify affected women within each family, all pedigrees were screened for transmitters (patients with an identified pathogenic mutation within the COL3A1 gene), likely transmitters (ascendants within one family branch with clinical features evocative of vascular EDS and/or characteristic arterial/digestive or obstetrical events, or a first degree ascendant without clinical features of vascular EDS, with one second degree ascendant with clinical features evocative of vascular EDS and/or characteristic arterial/digestive or obstetrical events) and unlikely transmitters (ascendants without clinical events evocative of vascular EDS, or without evocative arterial/digestive/obstetrical events, or having died of natural or identifiable non-vascular EDS related causes). In case of pedigrees with proven neomutations (both parents tested negative), only probands were considered for analysis.

### Definitions of maternal mortality

Maternal deaths were determined according to definitions of the 10^{th} revision of the International Classification of Diseases of the World Health Organization (Geneva 2004). Pregnancy-related deaths defined as the death of a woman while pregnant or within 42 days of termination of pregnancy, irrespective of the cause of death, were determined for probands, first and second degree ascendants. Late maternal deaths, defined by the death of a woman from direct or indirect obstetrical causes more than 42 days but less than one year after termination of pregnancy, were also considered, in order to increase the accuracy of the analysis. A global maternal mortality ratio was then determined, which encompassed pregnancy-related and late maternal mortality ratios. All mortality ratio calculations excluded accidental or incidental causes.

For purpose of clarity and according to common use, all mortality ratios are reported as mortality rates and are expressed per 100 live-births, with their respective 95% confidence interval (95%CI).

### Maternal morbidity study

Patients eligible for the maternal morbidity study had a first interview with a senior physician of our centre, during which relevant obstetrical data were collected. For each pregnancy, date and time of delivery were recorded, as well as term of pregnancy at birth, expressed in weeks of amenorrhea (WA). In case of preterm labour or premature rupture of membranes (PROM), gestational age of occurrence was noted. Ongoing treatments during pregnancy were recorded, especially betablockers (celiprolol). In case of anaesthesia, type of (epineural, general) and complications were collected (haematoma, leakage of spinal fluid, pneumothorax for general anaesthesia). Delivery was described as follows: type (vaginal, c-section), duration of labour for vaginal delivery and the use of forceps or episiotomy. Vascular complications were defined by the occurrence of arterial dissections and ruptures during pregnancy, delivery and during the post-partum period defined as 42 days after delivery. Haemorrhagic complications were defined by, either significant bleeding when reported as such by patients and/or medical records, either by bleeding requiring blood transfusion or post-partum haemorrhage. Bleeding events >1000ml with hemodynamic collapse are reported as haemorrhagic shock. Perineal damage was defined by the occurrence of a perineal tear related or not with episiotomy. Perineal injury was graded according to Sultan *et al.* [12], from grade I (limited defect of perineal skin), to grade IV (injury of the perineum involving the anal sphincter complex and epithelium) perineal tears.

After the first interview, all relevant obstetrical data, especially term of pregnancies and obstetrical complications (preterm labour, PROM, uterine rupture, haemorrhage) were independently checked for accuracy of diagnosis and staging by a senior obstetrician during a second interview, and hospital records of deliveries were checked, when available.

The study protocol was approved by the ethics committee of the French Society of Cardiology and all participants gave written informed consent.

### Newborn well-being

For each newborn, weight and height at birth were recorded and presented as a function of term of pregnancy, as previously described [13]. Health status at birth was assessed by the APGAR score [14] noted in the children's health record, and any significant event that occurred at birth was collected (respiratory failure, pneumothorax, significant verifiable birth defects).

### Statistical analysis

Descriptive statistics used numbers and percentages for qualitative variables, means and standard deviations for Gaussian quantitative variables, and medians and inter-quartile intervals for non Gaussian ones. Group comparisons were performed using Fisher exact tests for qualitative variables and two sample Wilcoxon tests for quantitative ones. Comparisons of observed distributions and theoretical ones were performed using exact tests and confidence intervals for proportions were computed using the Clopper-Pearson method. All tests were two-sided, with a p-value < 0.05 considered as significant. Computations were performed using the SAS v9 (SAS Institute Inc., Cary, North Carolina, USA) statistical package.

Reference charts for birth weight and size were constructed according to Battaglia *et al.* [13] with normal values based on approximately 57 000 newborn of the Paris (France) area as reported by Salomon *et al.*[15]*.*

### Results

### Maternal mortality

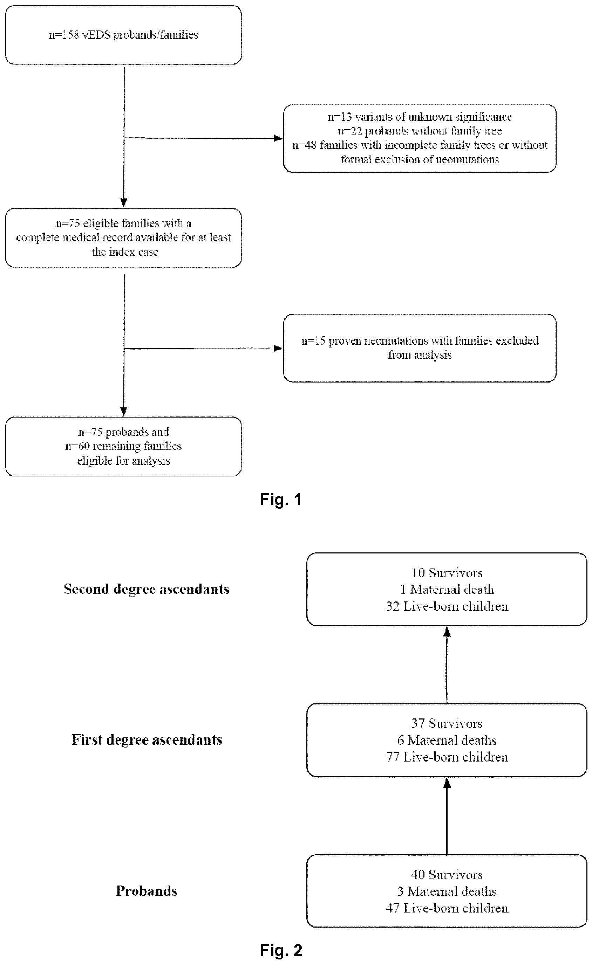

A total of n=158 families were screened for participation to the maternal mortality analysis (screening period from 2000 to 2014). After exclusion of incomplete pedigrees and of ascendants of patients with proven neomutations (Figure 1), n=75 probands and n=60 families remained eligible, of which n=59 families had a proband with an identified pathogenic COL3A1 variant. The n=1 remaining family without genetic testing was not excluded since the parturient had typical clinical features of vascular EDS (diagnosis was suspected as a result of these features documented photographically in a parturient that died during a first delivery by uterine rupture).

Amongst these n=60 families were n=98 parturients, being either probands (n=43), either first or second degree relatives (n=55), of which n=15 (27%) were genetically proven carriers of the mutation and n=40 (73%) were likely transmitters. These women totaled n=172 pregnancies (term pregnancies with live-born children: n=156; miscarriages: n=9; stillbirths: n=4; abortions n=3).

Distribution of live-born children, maternal deaths and surviving mothers by generation is shown in Figure 2. As expected, maternal death occurred mainly at delivery (n=7/10) rather than during pregnancy, either by arterial (n=4) or uterine rupture (n=3). One death occurred at 22 weeks of amenorrhea and one 7 months after delivery (see below), both by spontaneous intra-abdominal arterial rupture. The latter occurred spontaneously despite a programmed early c-section, medication by celiprolol and unremarkable arterial monitoring after the postpartum period. Characteristics of the deceased patients are shown in Table 1. Almost half of deaths occurred at first pregnancy, the other half was evenly distributed between the second and fourth pregnancies.

Maternal mortality rates for probands and ascendants are shown in Table 2. Despite marked differences, maternal mortality rates of 1^{st} and 2^{nd} degree ascendants did not differ significantly from probands (p=ns). Overall maternal mortality for all 3 generations was 6.4% 95%CI [3.1-11.5]. Interestingly, maternal death represented almost half (45.4%) of all causes of death in female patients aged 15 to 49 years in this cohort. As expected, maternal death occurred during delivery and the post-partum period, rather than during the course of pregnancy or within one year after delivery. Pregnancy-related mortality was significantly lower in probands when compared to ascendants, but most probands were still of child-bearing age at the time of the study, and thus have had fewer pregnancies. Amongst the n=43 probands, n=6 patients were delivered with specific protective measures and all survived the post-partum period.

### TABLE 1

**Table 1: Genetic and obstetrical characteristics of parturients (probands) with vascular Ehlers-Danlos syndrome, that died during pregnancy, delivery or within one year after delivery.**

| Patient | *COL3A 1* variant | | Number of pregnancies | Number of term pregnancies | Age at death (years) | Term | Complication |
|---|---|---|---|---|---|---|---|
| | DNA | Protein | | | | | |
| 1 | c.647 G>C | p.Gly216Ala | 3 | 2 | 30 | 24 WA | Arterial rupture (abdomen) |
| 2 | c.556 G>A | p.Gly186Ser | 3 | 3 | 27 | Delivery | Arterial rupture (abdomen) |
| 3 | c.2951 G>A | p.Gly984Glu | 1 | 0 | 40 | Delivery | Uterine rupture |
| 4 | c.2491 G>A | p.Gly831Asp | 2* | 2* | 34 | Delivery | Arterial rupture (abdomen) |
| 5 | c.2446-2 A>G | intronic variant | 4 | 3 | 27 | Delivery | Arterial rupture (abdomen) |
| 6 | c.575 G>A | p.Gly192Asp | 2 | 1 | 31 | Delivery | Uterine rupture |
| 7 | c.2553+1 G>A | intronic variant | 1 | 1 | 29 | Delivery | Aortic rupture (abdomen) |
| 8^{†} | | | 1 | 1 | 30 | Delivery | Uterine rupture |
| 9 | c.1953 A>G | p.Gly577Val | 1 | 1 | 30 | 7 months after delivery | Arterial rupture (abdomen) |
| 10 | c.970.G>A | p.Gly324Ser | 1 | 1 | 26 | 36 WA | Arterial rupture (abdomen) |

| | | | | | | | |
|---|---|---|---|---|---|---|---|
| Abbreviations: WA: weeks of amenorrhea. Legends: "*": twin pregnancy. †: patient highly suspect of vascular EDS (characteristic facial appearance, acrogeria, death at delivery consecutive to uterine rupture), in which genetic testing was not possible. She was not removed from analysis because of the rarity of maternal deaths and the risk of significantly underestimating maternal mortality calculations. | | | | | | | |

### TABLE 2

**Table 2: Pregnancy-related, late maternal and overall maternal mortality rates of vascular Ehlers-Danlos syndrome patients in probands and in confirmed or likely transmitters (ascendants) of the disease. Legend: n=9/10 (99%) of probands, n=13/43 (30.2%) of 1^{st} degree relatives and n=2/12 (16.7%) of 2^{nd} degree relatives had a confirmed pathogenic variant within the COL3A1 gene.**

| | Live born children | Maternal deaths (all) | Maternal deaths (pregnancy and peripartum) | Late maternal deaths (peripartum to 1 year) | Pregnancy-related mortality Ratio (95%CI) | Late maternal mortality ratio (95%CI) | Overall maternal mortality ratio (95%CI) |
|---|---|---|---|---|---|---|---|
| 2^{nd} degree ascendants | 32 | 1 | 1 | 0 | 3.1[0.1-16.2] | 0.0[0.0-0.0] | 3.1[0.1-16.2] |
| 1^{st} degree ascendants | 77 | 6 | 6 | 0 | 7.8[2.9-16.2] | 0.0[0.0-0.0] | 7.8[2.9-16.2] |
| Probands* | 47 | 3 | 2 | 1 | 4.3[0.5-14.5] | 2.1[0.1-11.3] | 6.4[1.0-20.3] |
| Probands + 1^{st} degree ascendants | 124 | 9 | 8 | 1 | 6.5[2.8-12.3] | 0.8[0.0-4.4] | 7.3[3.4-13.3] |
| Probands + 1^{st} + 2^{nd} degree ascendants | 156 | 10 | 9 | 1 | 5.8[2.7-10.7] | 0.6[0.0-3.5] | 6.4[3.1-11.5] |

### Maternal morbidity

Between November 2008 and July 2012, n=39 consecutive female patients with molecularly confirmed vascular EDS totalizing n=82 pregnancies were included in the obstetrical morbidity interview study (Table 3). These pregnancies divided into n=59 live-born children (term pregnancies), spontaneous abortions (n=12), voluntary terminations (n=7), medical abortion (n=1), still-births (n=2) and ectopic pregnancy (n=1).

One patient (3.2%) died seven months after delivery of spontaneous renal and hepatic artery rupture, despite a programmed and uneventful preterm c-section and normal arterial assessment before and after the post-partum period. Prevalence of arterial events (dissection, rupture) was low (5% of pregnancies) due to an obvious recruitment bias inherent to the study design (inclusion of surviving patients), and because a majority of patients (76%) was not diagnosed with vascular EDS at the time of their respective deliveries.

Prevalence of maternal non-lethal disease-related events including arterial accidents and uterine ruptures was 13%. Uterine rupture occurred repeatedly (n=2) in n=1 (2.5%) parturient, the first at 16 weeks of amenorrhea of a second pregnancy resulting in foetal death and the second at 38 weeks of amenorrhea of a third pregnancy, prompting urgent c-section. Other vEDS-related complications were n=1 colonic rupture during the post-partum period, n=1 bladder perforation during c-section and n=1 papillary muscle rupture causing acute mitral insufficiency requiring surgical repair (reported previously) [16].

One fifth of patients (n=9) had already presented a first major arterial (n=6) or digestive (n=3) event prior to their first pregnancy. Arterial accidents (n=12) were predominantly dissections (n=9) of the carotid, vertebral and iliac arteries. Spontaneous carotido-cavernous fistula occurred in two (5%) patients and one (2.5%) patient was diagnosed with an aneurysm of the splenic artery, which remained unremarkable throughout pregnancy and after the post-partum period. Digestive events were exclusively spontaneous colon perforations (n=3 patients). None of these patients experienced complications related to these events during their respective pregnancies, deliveries and post-partum periods.

Almost half of patients (49%) had two and only one (2.5%) patient had 3 pregnancies with live-born children.

### Course of pregnancy

For most women, pregnancy remained unremarkable. One pregnancy was interrupted by medical decision at 10 weeks of amenorrhea because of the occurrence of a carotid-cavernous fistula requiring treatment. A second pregnancy was interrupted by a spontaneous uterine rupture at 16 weeks of amenorrhea of a second pregnancy (see above). A second foetal death occurred at 24 weeks of amenorrhea related to eclampsia in a first pregnancy.

### Delivery

As a consequence of preterm labour and PROM (Table 3), n=19 (31%) of births were preterm, with a median term of 38 [35-39] weeks of amenorrhea. Deliveries were predominantly vaginal n=35 (57%) vs. n=19 (31%) by c-section. Conversion to c-section in the course of labour was necessary in n=3 (5%) deliveries.

Vaginal delivery was marked by the occurrence of perineal tears in n=19 cases (49%), half of which were third degree or more (Table 3). These tears occurred despite a widespread use of episiotomy and required repeat surgical repair in two patients. Despite important perineal morbidity, significant incontinence (urinary and faecal) occurred in only n=3 (8%) patients, persisting over 6 months after delivery in n=1 (3%) patient only.

Haemorrhagic complications in relation with uterine atony occurred predominantly during c-section (6/19, 32%), requiring blood transfusion in 5 deliveries out of 6, and haemostatic hysterectomy in one patient. Bleeding accidents were significantly associated with c-section when compared with vaginal delivery (Table 4). Bleeding during vaginal delivery was mainly related to direct tissular injury (tears), rather than uterine bleeding. Other indirect obstetrical complications were pulmonary embolism (n=1), deep (n=2) and superficial (n=1) vein thrombosis. Overall indirect obstetric event rate (haemorrhage and perineal complications) was 28/59 (47%).

No acute non-lethal arterial event (dissection or rupture) was diagnosed during any delivery. However, asymptomatic bilateral iliac artery dissection was evidenced during a systematic post-delivery vascular assessment in a 28 year old patient after vaginal delivery of a first pregnancy. Since a majority of patients gave birth without being diagnosed with vascular EDS, other undetected arterial events may have occurred. Conversely, in the n=12 (19%) deliveries (in n=11 parturients) in which specific protective measures were applied (see below), no silent arterial defect was identified after a systematic vascular assessment made between the 1^{st} and 3^{rd} months after delivery.

### TABLE 3

**Table 3: Maternal characteristics and obstetric outcome of n=39 parturients with molecularly confirmed vascular Ehlers-Danlos syndrome. * Defined as childbirth occurring less than 37 completed weeks or 259 days of gestation (ICD 10, Geneva 2004).**

| | **n (%) or Median [Q1-Q3]** |
|---|---|
| **Maternal characteristics** | |
| Age at delivery | 27 [24-30] |
| Age at genetic diagnosis | 38 [29-44] |
| vEDS diagnosed before delivery | 9 (24) |
| Total number of pregnancies | 82 |
| Type of *COL3A1* variant: | |
| Glycine substitutions | 23 (60) |
| Splice-site mutations/deletions/duplications | 12 (32) |
| C- or N-terminal mutations | 3 (8) |

| **Characteristics of pregnancies and deliveries (life and still-births; n=61)** | |
|---|---|
| Preterm labour | 23 (38) |
| Premature rupture of membranes | 12 (20) |
| Cervix incompetence (5 months) | 2 (3) |
| Preterm births* | 24 (40) |
| Term (weeks of amenorrhea) | 36 [33-37] |
| Arterial events | 2 (3) |
| Bleeding (all) | 12 (20) |
| Hemorrhage requiring blood transfusion | 6 (10) |
| Haemorrhagic shock | 4 (7) |

| **Perineal outcome in vEDS patients with vaginal deliveries (n=35)** | |
|---|---|
| Episiotomy (right mediolateral) | 24 (71) |
| Forceps delivery | 3 (9) |
| Perineal tears (all) | 19 (56) |
| 1^{st} degree | 4 (22) |
| 2^{nd} degree | 5 (28) |
| 3^{rd} degree | 8 (44) |
| 4^{th} degree | 1 (6) |
| Duration of pain (days) | 16.5 [5-40] |
| Incontinence | 3 (14) |
| urinary | 2 (67) |
| faecal | 1 (33) |
| Duration of incontinence | |
| from 3 to 6 months | 2 (67) |
| >6 months | 1 (33) |

### Anaesthesia

More than two thirds of patients (72%) had anaesthesia during delivery, dividing into epineural analgesia in n=27 (61%) and primary general anaesthesia in n=12 (27%) deliveries. Secondary conversion to general anaesthesia was necessary in n=5 (11%) cases. These conversions were mainly related to haemorrhagic and perineal complications requiring either haemostatic, either reparative surgery. No arterial, digestive, neurologic or respiratory complication formally related to anaesthesia was identified in any delivery.

### Programmed early c-section and prescription of celiprolol

In n=12 (20%) pregnancies of this cohort, diagnosis of maternal vascular EDS (n=11, 28%) was suspected (n=2) or genetically confirmed (n=9) at the time of delivery, with consequent obstetrical management (prescription of celiprolol 200mg BID in n=7 patients covering 7/12 pregnancies; preterm c-section in n=11/12 deliveries, of which 9 were scheduled). Notably, celiprolol was well tolerated, as no dose adjustment was necessary in any patient throughout pregnancy and the post-partum period, in comparison to the dosage prescribed prior to the beginning of each respective pregnancy. No arterial event (dissection, rupture), nor uterine rupture occurred in any patient treated with celiprolol, and all patients treated with celiprolol survived respective pregnancies and post-partum periods.

### Newborn well-being

Two foetal deaths occurred during pregnancy, the first after a spontaneous uterine rupture, and the second in a context of eclampsia (see above). Amongst the n=59 live-born, prematurity-related acute respiratory failure occurred in n=4 (7%) newborn, of which two required prolonged respiratory assistance. Other reported events were a bilateral pneumothorax occurring during respiratory assistance (n=1), pulmonary valve stenosis (n=1), renal atrophy (n=1), amniotic band complicated with toe constriction (n=1), epispadias (n=1) and umbilical cord rupture (n=1) at clamping.

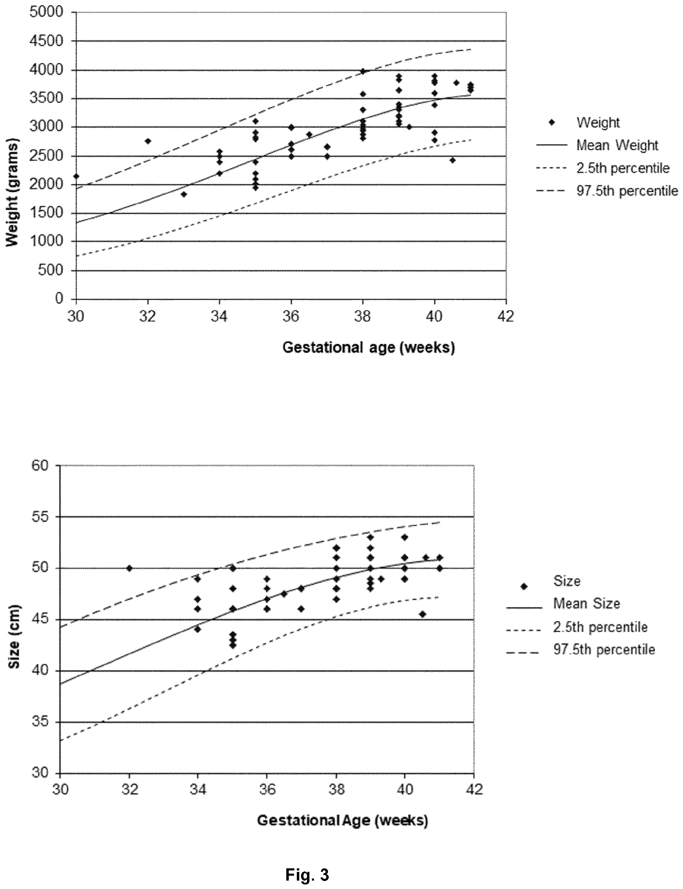

Despite significant prematurity, newborns did not show significant intrauterine growth retardation as illustrated in Figure 3 (all newborn; similar findings in male and female newborn studied separately, not shown), nor hypotonicity: Apgar score at 1 and 5 minutes were 8.8±2.7 and 9.3±2.1, respectively. The n=9 (15%) newborns for which positive genetic testing for a COL3A1 mutation was available at the time of the study, birth weight and size charts showed similar findings (not shown). Notably, none of the n=7 newborn of mothers who received celiprolol during pregnancy and delivery, did present neonatal hypoglycaemia, nor had other indications of adverse effects of indirect exposure to celiprolol during pregnancy.

Although the foregoing invention has been described in some detail by way of illustration and example for purposes of clarity of understanding, the descriptions and examples should not be construed as limiting the scope of the invention. The disclosure of all patent and scientific literature cited herein are expressly incorporated in their entirety by reference.

### REFERENCES

1. Pepin M, Schwarze U, Superti-Furga A, Byers PH: Clinical and genetic features of Ehlers-Danlos syndrome type IV, the vascular type. The New England journal of medicine 2000; 342: 673-80.
2. Frank M, Albuisson J, Ranque B, Golmard L, Mazzella JM, Bal-Theoleyre L et al: The type of variants at the COL3A1 gene associates with the phenotype and severity of vascular Ehlers-Danlos syndrome. Eur J Hum Genet 2015; 23: 1657-64.
3. Pearl W, Spicer M: Ehlers-Danlos syndrome. South Med J 1981; 74: 80-1.
4. Snyder RR, Gilstrap LC, Hauth JC: Ehlers-Danlos syndrome and pregnancy. Obstet Gynecol 1983; 61: 649-50.
5. Barabas AP: Vascular complications in the Ehlers-Danlos syndrome, with special reference to the "arterial type" or Sack's syndrome. J Cardiovasc Surg (Torino) 1972; 13: 160-7.
6. Rudd NL, Nimrod C, Holbrook KA, Byers PH: Pregnancy complications in type IV Ehlers-Danlos Syndrome. Lancet 1983; 1: 50-3.
7. Lurie S, Manor M, Hagay ZJ: The threat of type IV Ehlers-Danlos syndrome on maternal well-being during pregnancy: early delivery may make the difference. J Obstet Gynaecol 1998; 18: 245-8.
8. Murray ML, Pepin M, Peterson S, Byers PH: Pregnancy-related deaths and complications in women with vascular Ehlers-Danlos syndrome. Genet Med 2014; 16: 874-80.
9. Lind J, Wallenburg HC: Pregnancy and the Ehlers-Danlos syndrome: a retrospective study in a Dutch population. Acta Obstet Gynecol Scand 2002; 81: 293-300.
10. Hurst BS, Lange SS, Kullstam SM, Usadi RS, Matthews ML, Marshburn PB et al: Obstetric and gynecologic challenges in women with Ehlers-Danlos syndrome. Obstet Gynecol 2014; 123: 506-13.
11. Regitz-Zagrosek V, Blomstrom Lundqvist C, Borghi C, Cifkova R, Ferreira R, Foidart JM et al: ESC Guidelines on the management of cardiovascular diseases during pregnancy: the Task Force on the Management of Cardiovascular Diseases during Pregnancy of the European Society of Cardiology (ESC). Eur Heart J 2011; 32: 3147-97.
12. Sultan A: Obstetric perineal injury and anal incontinence. Clinical Risk 1999; 5: 193-6.
13. Battaglia FC, Lubchenco LO: A practical classification of newborn infants by weight and gestational age. J Pediatr 1967; 71: 159-63.
14. Apgar V: A proposal for a new method of evaluation of the newborn infant. Curr Res Anesth Analg 1953; 32: 260-7.
15. Salomon LJ, Bernard JP, de Stavola B, Kenward M, Ville Y: [Birth weight and size: charts and equations]. J Gynecol Obstet Biol Reprod (Paris) 2007; 36: 50-6.
16. Seve P, Dubreuil O, Farhat F, Plauchu H, Touboul P, Broussolle C: Acute mitral regurgitation caused by papillary muscle rupture in the immediate postpartum period revealing Ehlers-Danlos syndrome type IV. J Thorac Cardiovasc Surg 2005; 129: 680-1.
17. Taylor DJ, Wilcox I, Russell JK: Ehlers-Danlos syndrome during pregnancy: a case report and review of the literature. Obstet Gynecol Surv 1981; 36: 277-81.
18. Weinbaum PJ, Cassidy SB, Campbell WA, Rickles FR, Vintzileos AM, Nochimson DJ et al: Pregnancy management and successful outcome of Ehlers-Danlos syndrome type IV. Am J Perinatol 1987; 4: 134-7.
19. De Paepe A, Thaler B, Van Gijsegem M, Van Hoecke D, Matton M: Obstetrical problems in patients with Ehlers-Danlos syndrome type IV; a case report. Eur J Obstet Gynecol Reprod Biol 1989; 33: 189-93.
20. Sorokin Y, Johnson MP, Rogowski N, Richardson DA, Evans MI: Obstetric and gynecologic dysfunction in the Ehlers-Danlos syndrome. J Reprod Med 1994; 39: 281-4.

## Claims

1. Celiprolol or a pharmaceutically acceptable salt thereof, for use in treating vascular Ehlers-Danlos syndrome in a woman during pregnancy, during delivery, or during the peripartum period.

2. Celiprolol or a pharmaceutically acceptable salt thereof, for the use of claim 1, wherein celiprolol or a pharmaceutically acceptable salt thereof is administered to the woman a dosage of at least 100 mg per day.

3. Celiprolol or a pharmaceutically acceptable salt thereof, for the use of claim 1 or claim 2, wherein celiprolol or a pharmaceutically acceptable salt thereof is administered to the woman at a dosage ranging from 200 mg to 600 mg per day.

4. Celiprolol or a pharmaceutically acceptable salt thereof, for the use of any of claims 1 to 3, wherein celiprolol or a pharmaceutically acceptable salt thereof is administered to the woman at a daily dosage of 200 mg to 400 mg.

5. Celiprolol or a pharmaceutically acceptable salt thereof, for the use of any of claims 1 to 4, wherein celiprolol or a pharmaceutically acceptable salt thereof is administered twice daily.

6. Celiprolol or a pharmaceutically acceptable salt thereof, for the use of any of claims 1 to 5, wherein administration of celiprolol or a pharmaceutically acceptable salt thereof to the woman prevents or reduces the risk of cardiovascular and/or digestive and/or obstetrical events.

7. Celiprolol or a pharmaceutically acceptable salt thereof, for the use of any of claims 1 to 6, wherein administration of celiprolol or a pharmaceutically acceptable salt thereof to the woman prevents or reduces the risk of arterial dissection and rupture.

8. Celiprolol or a pharmaceutically acceptable salt thereof, for the use of any of claims 1 to 7, wherein administration of celiprolol or a pharmaceutically acceptable salt thereof to the woman prevents or reduces the risk of obstetrical accidents.

9. Celiprolol or a pharmaceutically acceptable salt thereof, for the use of any of claims 1 to 8, wherein administration of celiprolol or a pharmaceutically acceptable salt thereof to the woman prevents or reduces the risk of uterine rupture.

10. Celiprolol or a pharmaceutically acceptable salt thereof, for the use of any of claims 1 to 9, wherein administration of celiprolol or a pharmaceutically acceptable salt thereof to the woman reduces the risk of pregnancy-related death.

11. Celiprolol or a pharmaceutically acceptable salt thereof, for the use of any of claims 1 to 9, wherein administration of celiprolol or a pharmaceutically acceptable salt thereof to the woman reduces the risk of death of the woman during the first year following termination of pregnancy.

12. Celiprolol or a pharmaceutically acceptable salt thereof, for the use of any of claims 1 to 9, wherein administration of celiprolol or a pharmaceutically acceptable salt thereof to the woman reduces the risk of preterm birth.

13. A method for treating vascular Ehlers-Danlos syndrome in a woman during pregnancy, during delivery, or during the peripartum period, the method comprising administering celiprolol, or a pharmaceutically acceptable salt thereof, to the woman during pregnancy, during delivery, or during the peripartum period, thereby treating vascular Ehlers-Danlos syndrome in the woman.

14. The method of claim 1, wherein celiprolol, or a pharmaceutically acceptable salt thereof, is administered to the woman a dosage of at least 100 mg per day.

15. The method of claim 1, wherein celiprolol, or a pharmaceutically acceptable salt thereof, is administered to the woman at a dosage ranging from about 200 mg to about 600 mg per day.

16. The method of claim 1, wherein celiprolol, or a pharmaceutically acceptable salt thereof, is administered to the woman at a daily dosage of about 200 mg to about 400 mg.

17. The method of claim 1, wherein celiprolol, or the pharmaceutically acceptable salt thereof, is administered twice daily.

18. The method of claim 1, wherein the method prevents or reduces the risk of cardiovascular and/or digestive and/or obstetrical events.

19. The method of claim 1, wherein the method prevents or reduces the risk of arterial dissection and rupture.

20. The method of claim 1, wherein the method prevents or reduces the risk of obstetrical accidents.

21. The method of claim 1, wherein the method prevents or reduces the risk of uterine rupture.

22. The method of claim 1, wherein the method reduces the risk of pregnancy-related death.

23. The method of claim 1, wherein the method reduces the risk of death of the woman during the first year following termination of pregnancy.

24. The method of claim 1, wherein the method reduces the risk of preterm birth.
